# FASCICULE DE BREVET EUROPEEN

(11) **EP 0 814 703 B1**
(45) Date de publication et mention de la délivrance du brevet: **07.06.2000**
(21) Numéro de dépôt: 96905924.5
(22) Date de dépôt: 07.03.1996
(51) Int. Cl.: A61B 6/12, A61B 6/00, A61B 17/34

(54) **DISPOSITIF DE REPERAGE DE LESIONS SUSPECTES DU SEIN ET APPAREIL POUR SA MISE EN PLACE**
VORRICHTUNG ZUM LOKALISIEREN VON KRANKHAFTEN VERÄNDERUNGEN IN DER BRUST UND GERÄT ZUM POSITIONIEREN
DEVICE FOR LOCATING SUSPICIOUS BREAST LESIONS, AND APPARATUS FOR POSITIONING SAME

(30) Priorité: 08.03.1995 FR 9502722
(43) Date de publication de la demande: 07.01.1998
(73) Titulaire: Bouquet De La Joliniere, Jean, Henri, 92150 Suresnes (FR); WORCEL, Alexandre, F-75012 Paris (FR)
(72) Inventeur: Bouquet De La Joliniere, Jean, Henri, 92150 Suresnes (FR); WORCEL, Alexandre, F-75012 Paris (FR)
(74) Mandataire: L'Helgoualch, Jean
(86) Numéro de dépôt international: FR9600355
(87) Numéro de publication internationale: WO9627328

(56) Documents cités:
- WO-A-88/06864
- WO-A-90/15576
- US-A- 4 592 356
- US-A- 5 158 084
- US-A- 5 409 004

## Description

La présente invention concerne un dispositif utilisable en chirurgie, et plus particulièrement un dispositif de repérage de petites lésions suspectes du sein, telles que des microcalcifications, en vue de leur ablation optimisée par voie chirurgicale, ainsi qu'un appareil pour sa mise en place.

Le dépistage des cancers du sein s'effectue habituellement par mammographie, car cette technique permet de détecter des lésions de faibles dimensions telles que des microcalcifications. Il faut ensuite prélever des tissus de la glande mammaire pour les analyser et vérifier leur éventuel caractère cancéreux. Ce prélèvement se fait généralement par voie chirurgicale, ou par biopsie, ou encore par ponction au moyen d'une aiguille.

Habituellement, le praticien (chirurgien) repère les lésions suspectes sur les clichés mammographiques en prenant le mamelon du sein comme repère, et utilise les mesures effectuées de face et de profil pour déterminer approximativement la position de la lésion à prélever.

L'une des principales difficultés rencontrées par les praticiens est la localisation de la lésion détectée par la mammographie. En effet, la position du sein est très différente pendant la mammographie (de face et de profil), où la patiente est en position assise, le sein se trouvant comprimé entre deux plaques, et pendant la phase opératoire où la patiente est généralement en position allongée sur le dos, sur une table d'opération.

Il en résulte que les repères mammographiques ne correspondent pas précisément à la position réelle de la lésion lors de l'opération de prélèvement, que ce soit par voie chirurgicale, par biopsie ou par ponction. Or, les lésions constituées par des microcalcifications sont souvent des cancers débutants et non invasifs de faibles dimensions, dont le diamètre est de l'ordre de grandeur du millimètre, et n'excède pas 1 cm. Ces lésions ne présentent pas de consistance ni de coloration particulière. Leur repérage est donc difficile, même après mammographie sous plusieurs angles, par exemple de face et de profil.

En pratique, le chirurgien est amené à faire une estimation pour prélever un volume relativement important de tissu mammaire susceptible de contenir la lésion suspecte. Le prélèvement est ensuite contrôlé par radiographie pour vérifier la présence des microcalcifications, puis analysé sur place par un spécialiste (anapathologiste).

Malgré toutes les précautions prises lors de ces opérations, on constate très souvent, ultérieurement, qu'il reste des microcalcifications dans le sein. Ces microcalcifications sont repérées lors de mammographies effectuées quelques semaines après l'intervention décrite ci-dessus. Il est alors souvent nécessaire d'effectuer une nouvelle intervention.

En raison de ces difficultés, afin d'éviter de devoir procéder à plusieurs interventions successives, le chirurgien est obligé de prélever des quantités de tissus bien plus importantes qu'il ne serait nécessaire si les lésions détectées par mammographie pouvaient être ensuite repérées avec une bonne fiabilité et une précision satisfaisante. De plus, le chirurgien ne peut avoir la certitude que toutes les microcalcifications ont été prélevées, à moins d'élargir l'ablation de tissus de manière excessive, ce qui peut en outre occasionner un préjudice esthétique et psychologique important à la patiente.

Divers dispositifs ont été proposés pour tenter de remédier à ces difficultés. Par exemple, le brevet FR-A-2.660.545 décrit un appareillage de biopsie associée à une mammographie suivant deux angles à l'aide d'une tête rotative de prise d'image, destiné à localiser des lésions suspectes correspondant à des cancers débutants. Cependant, cet appareillage est complexe et son utilisation est peu commode pour la patiente.

Le brevet FR-A-2.666.217 décrit un dispositif de repérage stéréotaxique et de ponction d'une lésion de faibles dimensions, détectée par mammographie, au moyen d'un trocart ou d'une aiguille de cytoponction. Ce dispositif est conçu pour permettre la prise de deux images radiographiques sous deux angles différents, le sein de la patiente étant comprimé entre deux plateaux dont l'un comporte une zone d'accès au sein ainsi qu'un support de guidage vertical d'une aiguille de ponction, dont le mouvement est déterminé en fonction des résultats de la détection de la lésion par les clichés mammographiques. Ce dispositif impose d'immobiliser le sein de la patiente entre les deux plateaux, non seulement pendant la mammographie, mais aussi pendant l'opération de ponction et d'analyse du prélèvement, c'est-à-dire pendant une durée prolongée, ce qui nuit au confort de la patiente.

Le brevet US-A-5.158.084 décrit un dispositif de repérage de lésions dans un tissu, comprenant un fil flexible radio-opaque portant au moins deux marqueurs détectables par palpation, destinés à permettre au chirurgien de déterminer la position de l'extrémité du fil sans être obligé de le découvrir entièrement.

Le brevet US-A-4.592.356 décrit un dispositif d'ancrage pour biopsie comprenant une aiguille dont la pointe est en forme de double crochet et dont la tige comporte un filetage pour le positionnement d'une pièce de blocage contre la surface de la peau.

La demande de brevet WO-A-90/15576 décrit un appareil de mise en place d'un dispositif de repérage comprenant un moyen d'ancrage de forme hélicoïdale fixé sur une tige susceptible d'être mise en rotation à l'intérieur d'une aiguille creuse pour insérer le moyen d'ancrage dans les tissus.

La présente invention a pour objet un dispositif de repérage de lésion suspecte de faibles dimensions, telle qu'une microcalcification, permettant de repérer la lésion avec certitude et précision après détection dans le sein par mammographie, de manière à permettre une ponction, une biopsie ou une ablation chirurgicale ultérieurement sans risque d'imprécision de la localisation de la lésion.

L'invention a également pour objet un appareil pour la mise en place du dispositif de repérage ci-dessus.

Le dispositif de repérage de lésions de faibles dimensions détectées dans le sein par mammographie, selon la présente invention, se distingue en ce qu'il comprend un moyen d'ancrage constitué par un ou plusieurs éléments flexibles et élastiques susceptibles de se présenter sous une forme linéaire lors de la mise en place et de se déformer une fois mis en place dans les tissus mammaires, ainsi qu'au moins un fil de longueur au moins égale à la profondeur d'implantation du moyen d'ancrage dans le sein.

Le moyen d'ancrage et le fil qui lui est fixé peuvent avantageusement être réalisés en une matière radiotransparente, ou au contraire en matière radio-opaque selon les conditions souhaitées pour l'opération de ponction, de biopsie ou d'ablation chirurgicale ultérieure.

L'appareil pour la mise en place du dispositif de repérage selon la présente invention, comprend, en combinaison, une aiguille creuse ou trocart de longueur au moins égale à la profondeur d'implantation des moyens d'ancrage dans le sein, une tige s'engageant dans l'aiguille du trocart et repoussant les moyens d'ancrage susceptibles de passer dans le conduit du trocart, sur lesquels est fixé au moins un fil, ainsi qu'une pièce de blocage amovible disposée entre la base du trocart et la base de la tige.

Conformément à l'invention, la longueur de la tige est sensiblement égale à la longueur de l'aiguille, et la hauteur de la pièce de blocage amovible est de préférence approximativement égale à la longueur du moyen de blocage de forme linéaire en position repliée. Ainsi, la tige, une fois engagée dans l'aiguille creuse du trocart, laisse à l'extrémité de celle-ci un espace correspondant à la longueur du moyen d'ancrage. Lorsque la pièce de blocage est enlevée, la tige peut être engagée totalement dans l'aiguille du trocart, et elle repousse, hors du conduit de l'aiguille, le moyen d'ancrage qui peut se déployer en raison de la flexibilité et de l'élasticité du matériau utilisé pour sa fabrication. La base de la tige du trocart est pourvue d'un épaulement coopérant avec la pièce de blocage pour faciliter le maintien de celle-ci.

Comme indiqué ci-dessus, le moyen d'ancrage est réalisé en un matériau flexible et élastique, de préférence radiotransparent, susceptible de passer d'une forme repliée linéaire à une forme déployée occupant une configuration déterminée dans l'espace.

Sous forme repliée, le moyen d'ancrage est de forme globalement cylindrique, de diamètre sensiblement égal au diamètre interne du conduit de l'aiguille du trocart. Il peut être maintenu sous cette forme repliée linéaire par un simple manchon cylindrique, par exemple pour faciliter son introduction dans l'aiguille du trocart. Le diamètre total du moyen d'ancrage replié est compris entre 1 et 3 mm environ, et sa longueur est comprise entre 2 et 30 mm.

Sous forme déployée, le moyen d'ancrage peut être mono-brin curviligne, en forme d'arc de cercle ou de crochet, ou multibrin, chaque brin occupant une direction différente dans l'espace. La dimension maximale du moyen d'ancrage, une fois déployé, est d'environ 3 à 10 mm.

Le matériau utilisé pour la fabrication du moyen d'ancrage peut être choisi parmi les matériaux acceptables sur le plan chirurgical et compatibles avec les tissus mammaires dans lesquels ils se trouvent provisoirement implantés. De plus, ils doivent posséder une flexibilité et une élasticité suffisantes pour passer de la forme repliée à la forme déployée, comme indiqué ci-dessus. On choisit le matériau de préférence parmi les matériaux radiotransparents biocompatibles possédant les caractéristiques ci-dessus. On peut avantageusement choisir un matériau non ionisant pour faciliter certaines analyses (IRM).

On pourra utiliser par exemple des moyens d'ancrage réalisés en polyéthylène ou en un alliage nickel - titane à effet super-élastique, en polymère à mémoire de forme, ou en alliage organique ou métallique à mémoire de forme.

Par exemple, il peut être avantageux, conformément à la présente invention, d'utiliser un moyen d'ancrage constitué par un brin unique élastique à mémoire de forme, ou par plusieurs brins flexibles élastiques coaxiaux susceptibles de s'écarter les uns des autres.

Suivant une forme préférentielle de réalisation, la pointe de l'aiguille est biseautée. Il en résulte que le moyen d'ancrage, de préférence sous forme de brin unique, se replie progressivement dès que son extrémité distale se trouve au niveau du biseautage de la pointe de l'aiguille.

A la base du moyen d'ancrage, est fixé un fil en matériau qui peut être radiotransparent ou radio-opaque. Suivant une forme de réalisation, ce fil est fixé par une de ses extrémités à la base du moyen d'ancrage, et dans ce cas, seul un brin du fil est accessible. Suivant une variante, le fil passe dans un anneau prévu à la base du moyen d'ancrage, formant ainsi deux brins, et les deux extrémités du fil sont alors accessibles. Ce fil sert essentiellement à faciliter la localisation du moyen d'ancrage implanté dans les tissus mammaires.

Le fil peut être réalisé par exemple en polyester biocompatible d'un type disponible dans le commerce, par exemple en Ticron® ou en Dacron® (tresses de polyester siliconées).

Suivant une forme avantageuse de réalisation, l'appareil pour la mise en place du dispositif de repérage suivant l'invention comporte une bague de sertissage disposée à la base de l'aiguille du trocart, afin de permettre d'effectuer aisément la solidarisation du moyen d'ancrage et du fil par simple sertissage au moyen d'une pince ou d'un poinçon.

Le fonctionnement du dispositif selon la présente invention est extrêmement simple : le moyen d'ancrage, replié sous forme linéaire, est placé dans le conduit de l'aiguille du trocart, la tige est engagée dans le conduit du trocart de manière à repousser le moyen d'ancrage, et, simultanément, la pièce de blocage amovible est mise en place de manière à limiter l'engagement de la tige et empêcher qu'elle ne fasse sortir le moyen d'ancrage du conduit de l'aiguille; puis, en opérant sous contrôle radiographique, le praticien (radiologue) introduit d'une main le trocart dans le sein jusqu'à ce que la pointe soit au centre de la lésion, puis, de l'autre main, il retire la pièce de blocage amovible verrouillant le mécanisme, et, en appuyant sur la base de la tige formant piston, injecte la pièce d'ancrage préalablement introduite dans la tige du trocart. En sortant de l'extrémité du conduit, la pièce d'ancrage se déforme en raison de sa flexibilité et se fixe dans les tissus mammaires.

En pratique, le moyen d'ancrage et le fil qui lui est fixé, ainsi que la tige et le moyen de blocage, sont mis en place dans l'aiguille du trocart, avant l'utilisation de l'appareil de l'invention, le moyen d'ancrage se trouvant en bout d'aiguille, prêt à être injecté dans les tissus mammaires par le praticien.

Le trocart et la tige sont ensuite enlevés et ne reste plus dans le sein que le moyen d'ancrage et le fil en matière radiotransparente dont l'extrémité dépassant hors du sein peut être coupée. Un pansement stérile et hermétique peut ensuite être mis en place.

Ce moyen d'ancrage servant de repère peut rester en place dans le sein pendant quelques jours, jusqu'à l'intervention chirurgicale d'ablation de la lésion. Ainsi, le repérage de la lésion dans le sein, pour permettre son ablation, ne dépend plus de la position du sein. L'ablation peut alors être limitée précisément à la lésion, ce qui simplifie l'opération chirurgicale. Le moyen d'ancrage et de repérage radiotransparent est enlevé aisément lors de l'opération chirurgicale d'ablation de la lésion.

Lorsque le moyen d'ancrage et le fil sont réalisés en matière radiotransparente, il est possible de procéder sans difficulté à des mammographies complémentaires, si cela s'avère nécessaire.

De plus, le dispositif conforme à la présente invention est utilisable sans qu'il soit nécessaire de recourir à un appareillage complexe et coûteux autre que l'appareil de mammographie, qui peut être d'un type usuel et qu'il n'est pas nécessaire de modifier.

Contrairement aux dispositifs classiques de la technique, le dispositif de l'invention peut être manipulé d'une seule main, dès que la pièce de blocage a été enlevée, ce qui facilite considérablement la mise en place précise du moyen d'ancrage près de la lésion.

Enfin, le dispositif de l'invention peut être appliqué pour le repérage d'autres lésions susceptibles d'être détectées dans d'autres tissus mous.

Les exemples décrits ci-après illustrent l'invention plus en détail, sans en limiter la portée, en référence aux dessins annexés, qui représentent :

Figure 1 : une vue en perspective d'un moyen d'ancrage en matériau super-élastique, conforme à l'invention.

Figure 2 : une vue en perspective d'un autre moyen d'ancrage conforme à l'invention.

Figure 3 : une vue d'une variante du moyen d'ancrage de la Figure 2.

Figure 4 : une vue en perspective d'une trocart équipé d'une pièce de blocage amovible, pour l'implantation des moyens d'ancrage.

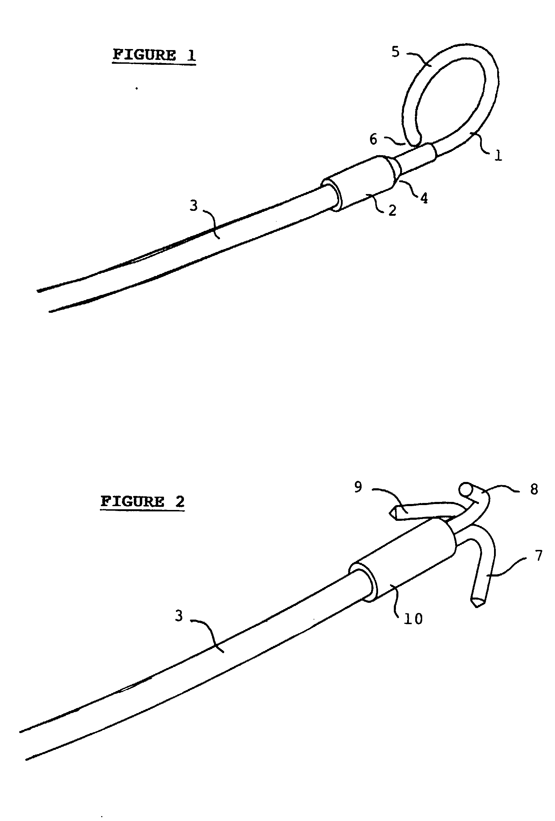

Comme représenté sur la Figure 1, le moyen d'ancrage est constitué par un brin (1) occupant une forme curviligne lorsqu'il n'est pas maintenu dans l'aiguille du trocart. A la base du moyen d'ancrage (1), se trouve un manchon (2) dans lequel est serti un fil (3) sortant vers l'arrière.

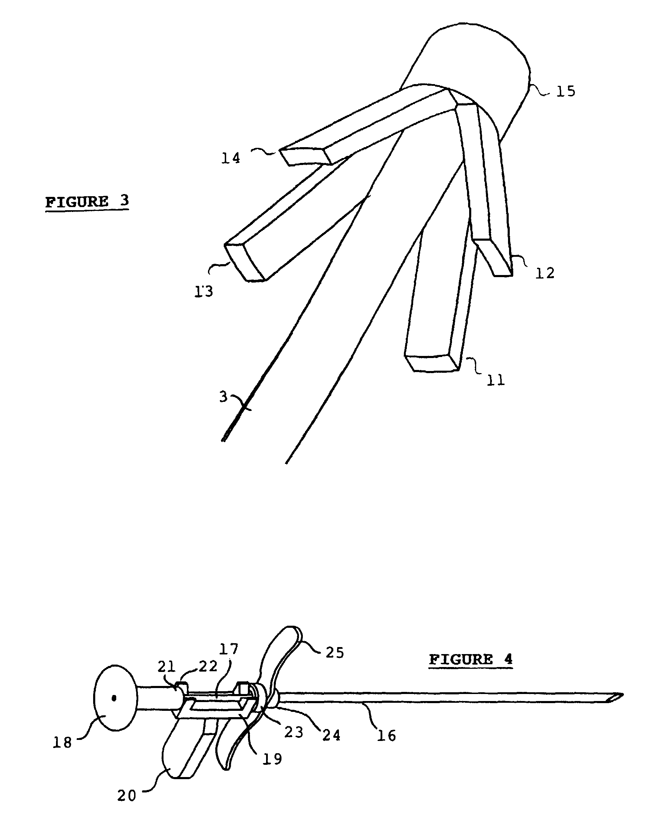

Le manchon (2) comporte une tête tronconique (5) facilitant sa mise en place à la base de l'aiguille du trocart représenté sur la Figure 4.

Le brin (1) comporte une partie (5) en arc de cercle d'au moins 270°, et une extrémité (6) en pointe facilitant sa pénétration dans les tissus mammaires. Il est réalisé en alliage nickel - titane à effet super-élastique. Dans le conduit de l'aiguille du trocart, il présente une forme linéaire adaptée à la forme intérieure du conduit, et, en sortant de l'aiguille, lorsqu'il est repoussé par la tige du trocart, il se replie en arc de cercle de 8 mm de diamètre environ. Cette forme offre une résistance suffisante pour que le moyen d'ancrage ne se déplace pas dans le tissu mammaire en fonction des mouvements du corps de la patiente.

Le moyen d'ancrage représenté sur la figure 2 comporte trois brins (7, 8 et 9) flexibles élastiques rattachés par leur base, au moyen d'un sertissage dans le manchon (10).

Ces trois brins, une fois déployés à la sortie de l'aiguille du trocart, ont une forme de crochet, les plans contenant chacun de ces crochets faisant entre eux un angle d'environ 120°.

Le fil (3) sort à l'arrière du manchon de sertissage (10) à la base des trois brins (7, 8 et 9).

Le moyen d'ancrage représenté sur la Figure 3 est une variante de celui de la Figure 2, comportant quatre languettes (11, 12, 13 et 14) réunies par leur sommet solidaire d'un capuchon (15), et susceptibles de s'écarter les unes des autres en pivotant par rapport à leur sommet.

Le diamètre du capuchon (15) correspond au diamètre intérieur du conduit de l'aiguille du trocart. Sa forme est profilée pour favoriser la pénétration dans les tissus.

En position normale, les bases des quatre languettes sont écartées les unes des autres. La flexibilité et l'élasticité de la matière utilisée permet de les rapprocher pour les introduire dans le conduit de l'aiguille, et dès la sortie de l'aiguille, elles s'écartent à nouveau en raison de leur élasticité.

Le fil (3), représenté sur la Figure 3, est rattaché à la base des languettes, comme dans l'exemple précédent, par sertissage.

L'appareil dans le capuchon (15) de mise en place des moyens d'ancrage est représenté sur la Figure 4.

Il est constitué d'un trocart comportant une aiguille (16) creuse dans laquelle peut s'engager une tige (17) que l'on enfonce dans le conduit de l'aiguille en appuyant sur le poussoir (18) jusqu'à ce qu'il vienne en butée contre la pièce de blocage amovible (19) qui peut être mise en place et enlevée en agissant sur la poignée (20). Un épaulement (21) sur la base de la tige (17) facilite la mise en place de la pièce de blocage (19) en coopérant avec les faces d'appui (22).

Lorsque la pièce de blocage (19) est enlevée, on peut appuyer sur le poussoir (18) pour faire pénétrer la tige (17) dans le conduit de l'aiguille (16) jusqu'à ce que l'épaulement (21) vienne en contact avec le disque (23) de la bague de sertissage (24).

La poignée (25) solidaire de l'aiguille (16) facilite la manipulation de l'ensemble.

## Revendications

1. Dispositif de repérage de lésions de faibles dimensions détectées dans le sein par mammographie pour faciliter une ponction, une biopsie ou une ablation chirurgicale, caractérisé en ce qu'il comprend un moyen d'ancrage (1) constitué par un ou plusieurs éléments flexibles et élastiques susceptibles de se présenter sous une forme linéaire lors de la mise en place et de se déformer une fois mis en place, ainsi qu'au moins un fil (3) de longueur au moins égale à la profondeur d'implantation du moyen d'ancrage dans le sein.

2. Dispositif selon la revendication 1, caractérisé en ce que les moyens d'ancrage (1) et le fil (3) sont en matière radiotransparente.

3. Dispositif selon la revendication 1, caractérisé en ce que les moyens d'ancrage (1) et le fil (3) sont en matière radio-opaque.

4. Dispositif selon l'une quelconque des revendications précédentes, caractérisé en ce que le moyen d'ancrage (1) est constitué par un brin unique (5) élastique à mémoire de forme.

5. Dispositif selon l'une quelconque des revendications 1 et 3, caractérisé en ce que le moyen d'ancrage (1) est constitué par plusieurs brins (7,8, 9) flexibles élastiques coaxiaux susceptibles de s'écarter les uns des autres.

6. Dispositif selon l'une quelconque des revendications précédentes, caractérisé en ce que le moyen d'ancrage (1) est maintenu en position repliée linéaire par un manchon cylindrique (10).

7. Appareil pour la mise en place d'un dispositif de repérage selon l'une quelconque des revendications précédentes, comprenant une aiguille creuse (16) ou trocart dans laquelle les moyens d'ancrage (1) sont susceptibles de passer, de longueur au moins égale à la profondeur d'implantation des moyens d'ancrage dans le sein, caractérisé en ce qu'il comprend une tige (17) s'engageant dans le trocart (16) et repoussant les moyens d'ancrage (1), sur lesquels est fixé au moins un fil, et une pièce de blocage amovible (19) disposée entre la base du trocart et la base de la tige.

8. Appareil selon la revendication 7, caractérisé en ce que la base de la tige du trocart est pourvue d'un épaulement (21) coopérant avec la pièce de blocage (19) pour faciliter le maintien de celle-ci.

9. Appareil selon l'une quelconque des revendications 7 et 8, caractérisé en ce que la longueur de la tige (17) est sensiblement égale à la longueur de l'aiguille (16) et la hauteur de la pièce de blocage (19) est approximativement égale à la longueur du moyen d'ancrage en position repliée linéaire.

10. Appareil selon l'une quelconque des revendications 7 et 8, caractérisé en ce qu'il comporte une bague de sertissage (24) disposée à la base de l'aiguille (16).

11. Appareil selon l'une quelconque des revendications 7 à 9, caractérisé en ce que la pointe de l'aiguille (16) est biseautée.

## Patentansprüche

1. Vorrichtung zur Ortung von Läsionen mit geringen Abmessungen, die durch Mammographie in der Brust detektiert worden sind, um eine Punktion, eine Biopsie oder eine chirurgische Ablation zu erleichtern, dadurch gekennzeichnet, dass sie ein Verankerungsmittel (1), das aus einem oder mehreren flexiblen und elastischen Elementen besteht, die beim Anbringen in linearer Form vorliegen und sich nach der Anbringung verformen können, sowie zumindest einen Draht (3) umfasst, dessen Länge zumindest gleich groß wie die Implantationstiefe des Verankerungsmittels in der Brust ist.

2. Vorrichtung nach Anspruch 1, dadurch gekennzeichnet, dass die Verankerungsmittel (1) und der Draht (3) aus strahlendurchlässigem Material bestehen.

3. Vorrichtung nach Anspruch 1, dadurch gekennzeichnet, dass die Verankerungsmittel (1) und der Draht (3) aus strahlenundurchlässigem Material bestehen.

4. Vorrichtung nach einem der vorangegangenen Ansprüche, dadurch gekennzeichnet, dass das Verankerungsmittel (1) aus einem einzigen elastischen Strang (5) mit Form-Memoryeffekt besteht.

5. Vorrichtung nach einem der Ansprüche 1 bis 3, dadurch gekennzeichnet, dass das Verankerungsmittel (1) aus mehreren koaxialen flexiblen elastischen Strängen (7, 8, 9) besteht, die sich voneinander weg spreizen können.

6. Vorrichtung nach einem der vorangegangenen Ansprüche, dadurch gekennzeichnet, dass das Verankerungsmittel (1) durch eine zylindrische Hülse (10) in linearer eingezogener Position gehalten wird.

7. Gerät für die Anbringung einer Vorrichtung zur Ortung nach einem der vorangegangenen Ansprüche, das eine Hohlnadel (16) oder einen Trokar umfasst, durch die bzw. den die Verankerungsmittel (1) hindurchgehen können, deren bzw. dessen Länge zumindest gleich der Implantationstiefe der Verankerungsmittel in der Brust ist, dadurch gekennzeichnet, dass es einen Schaft (17), der in den Trokar (16) eingreift und die Verankerungsmittel (1) zurückdrängt, auf denen zumindest ein Draht befestigt ist, sowie ein abnehmbares Verriegelungsstück (19) umfasst, das zwischen der Basis des Trokars und der Basis des Schafts angeordnet ist.

8. Gerät nach Anspruch 7, dadurch gekennzeichnet, dass die Basis des Schafts des Trokars mit einem Ansatz (21) versehen ist, der mit dem Verriegelungsstück (19) zusammenwirkt, um dessen Halten zu erleichtern.

9. Gerät nach einem der Ansprüche 7 und 8, dadurch gekennzeichnet, dass die Länge des Schafts (17) im Wesentlichen gleich der Länge der Nadel (16) ist und die Höhe des Verriegelungsstücks (19) in etwa gleich der Länge des Verankerungsmittels in linearer eingezogener Position ist.

10. Gerät nach einem der Ansprüche 7 und 8, dadurch gekennzeichnet, dass es einen Schrumpfring (24) umfasst, der an der Basis der Nadel (16) angeordnet ist.

11. Gerät nach einem der Ansprüche 7 bis 9, dadurch gekennzeichnet, dass die Spitze der Nadel (16) angefast ist.

## Claims

1. Device for pinpointing lesions of small dimensions detected in the breast by mammography in order to facilitate puncturing, biopsy or surgical ablation, characterized in that it comprises an anchoring means (1) consisting of one or more flexible and elastic elements which are capable of assuming a linear form during positioning and of deforming once in place, as well as at least one wire (3) of a length which is at least equal to the depth of implantation of the anchoring means within the breast.

2. Device according to Claim 1, characterized in that the anchoring means (1) and the wire (3) are made of radioparent material.

3. Device according to Claim 1, characterized in that the anchoring means (1) and the wire (3) are made of radiopaque material.

4. Device according to any one of the preceding claims, characterized in that the anchoring means (1) consists of a single elastic strand (5) with shape memory.

5. Device according to either of Claims 1 and 3, characterized in that the anchoring means (1) consists of several coaxial, elastic and flexible strands (7, 8, 9) which are capable of spacing apart from one another.

6. Device according to any one of the preceding claims, characterized in that the anchoring means (1) is held in the linear, folded-up position by a cylindrical sleeve (10).

7. Apparatus for positioning a pinpointing device according to any one of the preceding claims, comprising a hollow needle (16) or trocar into which the anchoring means (1) is capable of passing, and of a length at least equal to the depth of implantation of the anchoring means within the breast, characterized in that it comprises a rod (17) engaging in the trocar (16) and pushing back the anchoring means (1), on which means at least one wire is fixed, and a removable blocking piece (19) disposed between the base of the trocar and the base of the rod.

8. Apparatus according to Claim 7, characterized in that the base of the rod of the trocar is provided with a shoulder (21) cooperating with the blocking piece (19) to make it easier to hold the latter.

9. Apparatus according to either of Claims 7 and 8, characterized in that the length of the rod (17) is substantially equal to the length of the needle (16), and the height of the blocking piece (19) is approximately equal to the length of the anchoring means in the linear, folded-up position.

10. Apparatus according to either of Claims 7 and 8, characterized in that it includes a crimping ring (24) disposed at the base of the needle (16).

11. Apparatus according to any one of Claims 7 to 9, characterized in that the point of the needle (16) is beveled.
